# EUROPEAN PATENT APPLICATION

(11) **EP 2 386 329 A1**
(43) Date of publication of application: **16.11.2011**
(21) Application number: 10382115.3
(22) Date of filing: 11.05.2010
(51) Int. Cl.: A61N 5/06

(54) **Apparatus for the skin treatment with visible light**

(71) Applicant: S.O.R. INTERNACIONAL, S.A., 08192 Sant Quirze Del Vallès (ES)
(72) Inventor: Sánchez Soriano, Manuel, 08192, SANT QUIRZE DEL VALLÈS (ES); Amat Genis, Alberto, 08192, SANT QUIRZE DEL VALLÈS (ES); Vadillo Pastor, Juan Ramón, 08192, SANT QUIRZE DEL VALLÈS (ES)
(74) Representative: Manresa Val, Manuel

(57) **Abstract**

Apparatus for treating skin with visible light for application to the face, body or both, of the type comprising a plurality of light sources (3), connected to power supply means, characterized in that said light sources are light emitting diodes (LEDs) (3) with a visible monochromatic violet light wave length between 401 and 420 nm, and said power supply means comprise switched control elements under current that maximize the energy efficiency and service life of the assembly.

## Description

Apparatus for treating skin with visible light for application to the face, body or both, of the type comprising a plurality of light sources, connected to power supply means, characterized in that said light sources are light emitting diodes (LEDs) with a visible monochromatic violet light wave length of between 401 and 420 nm, and said power supply means comprise switched control elements under current that maximise the energy efficiency and service life of the assembly.

### BACKGROUND TO THE INVENTION

The appearance of tanning equipment marked the start of a significant stage of the well-being and beauty care business.

At present, existing tanning equipment consists of UV light radiation, mainly in the range of UVA (320 - 400 nm) with a very small contribution of UVB (280 - 320 nm). The property of these two types of UV light is to stimulate melanin synthesis, in other words, to darken the skin. However, the World Health Organization warns that uncontrolled and extended exposure to UV rays can cause skin cancer and premature skin ageing.

Furthermore, some manufacturers are searching for technical solutions to minimise risks, using new UVA emission means, such as for example, light emitting diodes (LEDs).

So, in the state of the art, US Patent No. 6.861.658 "Skin tanning and light therapy incorporation light emitting diodes" is known. The object of this patent of invention is to perfect a tanning booth wherein light emitting diodes (LEDs) are used. Essentially, at least one light emitting diode (LEDs) must emit only the UV light. The electric current control means supply power to the LEDs and maintain the current flows calibrated and constant throughout the service life of the LED. The LEDs are contained in a flexible structure.

### BRIEF DESCRIPTION OF THE PATENT

This invention is a major development in the aesthetics, aesthetical medicine and the dermatology sector.

The inventors are known in the sector for having created several registers relating to electroaesthetical and electromedicinal apparatuses.

These inventors have conducted a study on the effects of exposing skin to light that is part of the visible spectrum, and not UV light.

Taking into account the known risks of UV light on skin, the inventors have carried out an experiment on wave lengths close to a non-visible spectrum, between 401 nm and 420 nm, which corresponds to a monochromatic violet light spectrum that is already visible.

To this end, various tests have been carried out on exposing areas on the back of the hands of various people with phototypes from II to IV, to said light.

The light source was made up from 405 nm LED diodes, in the form of panels light emitting, which had to be built purposely for this reason as they did not exist on the market.

The results showed a darkening in the colour of the skin as a result of the treatment with a panel of light emitting diodes (LEDs) at 405 nm, which was verified in two different ways: i) with equipment that emits and measures the light reflected from the skin using a photodiode, giving more or less high value depending on the colour of the skin in question; and ii) with a visual scale of colours. In both cases, the initial values (before exposure to the preferred source of LEDs at 405 nm) and the final values (post-exposure to light at 405 nm) were observed. The result of both measurements was statistically significant in terms of the variation of skin colour.

LED technology was considered because it is cheaper and more efficient, and provides more durability and safety on the permanence of lighting.

Moreover, it was necessary to build an apparatus with a large intense radiation area with a predetermined wave length. In order to build a large-scale panel, it was divided into independent modules that are controlled independently. Also, building an apparatus with a large emission surface implies high energy consumption, and so, in order to minimize energy dissipation in auxiliary elements, the switched control system was used, which is explained in more detail in the particular embodiment.

Although in the section of the particular embodiment of the application of the violet light source using LED emitting in the range of 401 - 420 nm, said application is limited to tanning the skin, the inventors have ascertained that it can be extended to the therapies used for certain skin conditions. These applications include, but not exclusively, treating acne (415-419 nm) and atopic dermatitis (401-408 nm) by applying the light directly.

Another possible treatment within the light range 401 - 420 nm is the application of said light together with a photosensitive element so as to produce cell toxicity and eliminate certain areas or skin lesions. This application is called photodynamic therapy, and it is produced preferably in the spectrum of 415 to 419 nm. With this technique it is possible to effectively treat oral mucous dermatitis, certain types of skin cancer, actinic keratosis and signs of skin photoageing, as well as other skin conditions.

The object of this invention is an apparatus for treating skin with visible light for application to the face, body or both, of the type comprising a plurality of light sources, connected to a power supply means characterized in that said light sources are light emitting diodes (LEDs) with a visible monochromatic violet light wave length between 401 and 420 nm, and said power supply means comprise switched control elements under current, which maximise the energy efficiency and service life of the assembly.

### BRIEF DESCRIPTION OF THE DRAWINGS

In order to facilitate the explanation, two sheets of drawings are attached to this invention, which illustrate a practical embodiment thereof, which is provided as a non-limiting example of the scope of this invention:
- Figure 1 shows the construction of a panel using a plurality of modules;
- Figure 2 is a diagram of the basic structure of the LEDs current source in a module.

### CONCRETE EMBODIMENT OF THE UTILITY MODEL

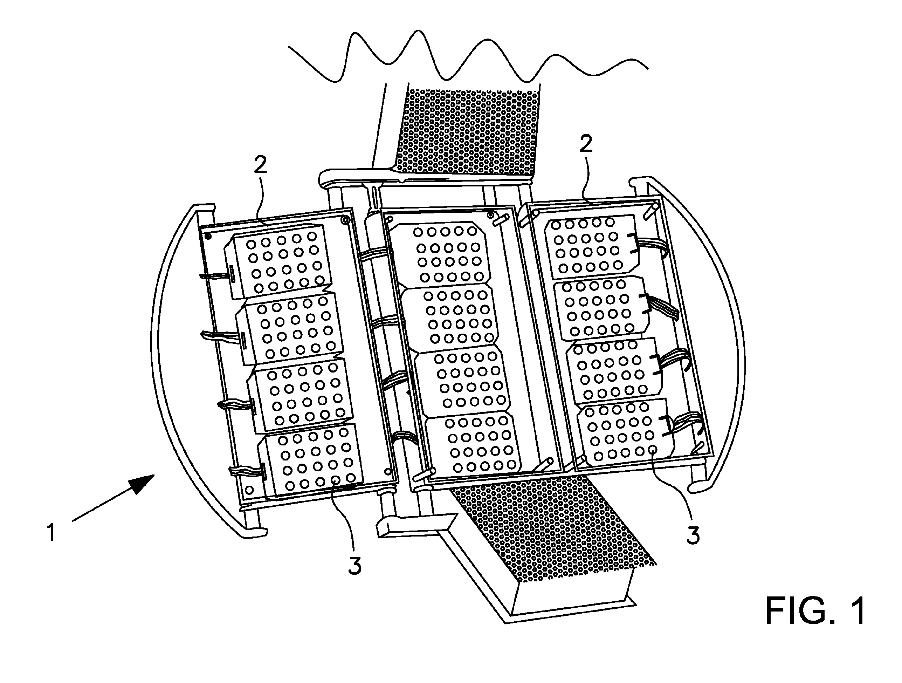

Figure 1 shows a LED light panel 1 made up of three modules 2, each one having a plurality of LEDs 3.

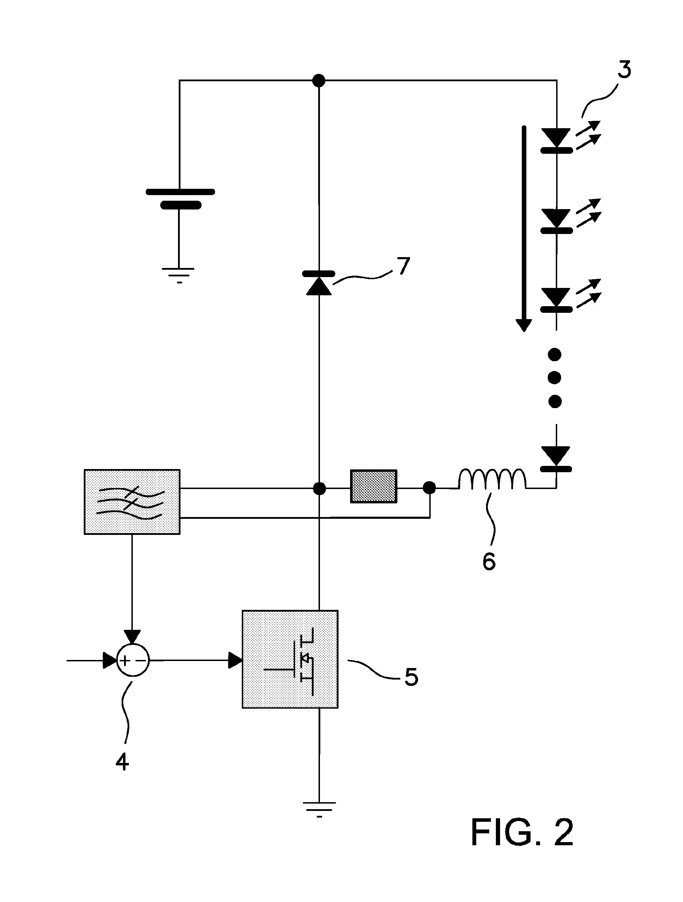

Figure 2 shows an operating circuit of the current source for a LEDs module.

The circuit includes a plurality of serially connected LEDs 3, a power supply 4, which can be shared with other modules, a MOSFET transistor 5, a current measurement resistance 6 connected between the MOSFET transistor 5 and the serial LEDs 3 and a recirculating diode 7.

Said LEDs 3 emit light in a wave length of between 400 - 420 nm, although it has been observed that the spectrum area for obtaining better tanning results, without the dangerous side effects of UV radiation, is 405 nm.

So, the system switches the current through a MOSFET transistor 5.

Over a period of time, said MOSFET transistor 5 conducts a current to ground, while also making the current pass through resistance 6. This way it is possible to reach a certain level of objective current. Once said level of objective current is reached, said MOSFET transistor 5, stops conducting current.

The current, then, is forced to pass through recirculating diode 7. This leads to a gradual reduction in the current until it is below a predetermined threshold, at which point, the MOSFET transistor 5 starts to conduct the current again. This operation is repeated automatically.

Through this structure it is possible to achieve high efficiency and a prolonged service life of the LEDs.

This patent of invention describes a new apparatus for tanning with visible light. The tanning apparatus can be for application to the face, body or both. The examples mentioned herein are non-limiting with respect to the invention, and therefore said invention can have different applications and/or adaptations, all included within the scope of the following claims.

## Claims

1. Apparatus for treating skin with visible light for application to the face, body or both, of the type comprising a plurality of sources of light (3), connected to a power supply **characterized in that**:
- said light sources are light emitting diodes (LEDs) (3) with a visible monochromatic violet light wave length between 401 and 420 nm, and
- said power supply means comprise switched control elements under current that maximise the energy efficiency and service life of the assembly.

2. Apparatus according to claim 1, **characterized in that** said light sources (3) are divided into modules (2) and are controlled independently.

3. Apparatus according to claims 1 or 2, **characterized in that** the wave length of the LEDs (3) varies between 401 and 410 nm.

4. Apparatus according to claim 3, **characterized in that** the wave length of the LEDs (3) is 405 nm.

5. Apparatus for tanning according to claim 1, **characterized in that** one of the control elements is a MOSFET transistor (5).

6. Apparatus for tanning according to claim 5, **characterized in that** the power source comprises at least one recirculating diode (7).
